# EUROPEAN PATENT SPECIFICATION

(11) **EP 4 431 160 B1**
(45) Date of publication and mention of the grant of the patent: **19.08.2026**
(21) Application number: 24163052.4
(22) Date of filing: 12.03.2024
(51) Int. Cl.: A61Q 19/00, A61Q 17/00, A61K 8/36, A61K 8/69, A61K 8/03, A61P 17/10

(54) **BIPHASIC COMPOSITION FOR TOPICAL USE**
ZWEIPHASIGE ZUSAMMENSETZUNG ZUR TOPISCHEN VERWENDUNG
COMPOSITION BIPHASIQUE POUR UTILISATION TOPIQUE

(30) Priority: 17.03.2023 IT 202300005049
(43) Date of publication of application: 18.09.2024
(73) Proprietor: VIDA S.R.L., 56040 Crespina Lorenzana (PI) (IT)
(72) Inventor: SCAFFAI, Dario, 57128 Livorno (IT)
(74) Representative: Firmati, Leonardo

(56) References cited:
- CN-A- 109 481 328
- US-A1- 2014 037 561
- US-A1- 2018 228 715

## Description

### TECHNICAL FIELD

This invention relates to a composition for topical use, in particular for the treatment of skin imperfections, as defined in the appended claims.

### BACKGROUND ART

Acne is an inflammation caused by the excessive functioning of the sebaceous glands that surround the base of hairs (follicles) and produce sebum, a fatty substance that protects the skin. If excessive sebum is produced it deposits and obstructs the follicle and forms plugs, the so-called 'blackheads'. This condition promotes the proliferation of normally harmless bacteria, present on the skin, which can cause inflammation and infection inside the obstructed follicle, giving rise to papules and pustules.

In the most serious cases, retinoid-based drugs are currently proposed for systemic use, or isotretinoin-based antibacterial products. These drugs, acting at a systemic level, may cause side effects in susceptible individuals.

Alternatively, in less severe cases, exfoliating peeling that leads to removal of the surface epidermal layer is recommended to promote stimulation of the skin and cell renewal.

Some of the most effective peels are trichloroacetic acid (TCA) peels. See e.g. US 2014/037561 A1 (KULESZA JOHN E [US] ET AL) 6 February 2014 (2014-02-06), US 2018/228715 A1 (SOMERVILLE KATE [US] ET AL) 16 August 2018 (2018-08-16), CN 109 481 328 A (GUANGZHOU WEILING BIO TECH CO LTD) 19 March 2019 (2019-03-19).

The action mechanism of the chemical peeling currently on the market is based solely on the exfoliation caused by an active acid ingredient, neglecting the other factors which contribute to the formation of acne and its blemishes, such as, for example, bacterial proliferation.

In this context, a first aim of the invention is to provide an exfoliating composition for topical use, in particular for the treatment of acne, which associates with an active acid ingredient at least one active ingredient which is able to act on the further factors which favour the formation of acne-related blemishes.

A further aim of the invention is to provide an exfoliating composition for topical use, in particular for the treatment of acne, which is highly tolerable for the skin and with a low inflammatory power.

### SUMMARY OF THE INVENTION

According to the invention, these aims and others are achieved by a biphasic composition for topical use as described in independent claim 1 or in any of the claims dependent thereon.

This invention also relates to a method for preparing the biphasic composition for topical use, see the appended claims.

### DETAILED DESCRIPTION OF THE INVENTION

In the context of the present invention, the term "perfluorocarbon" refers to a chemical compound consisting only of fluorine and carbon.

In the context of this invention, the percentages are expressed in weight, unless otherwise indicated.

In the context of this invention, the sum of the quantities of components included in a composition or mixture is equal to 100% by weight, unless otherwise indicated.

According to a first aspect, this invention relates to a biphasic composition for topical use comprising an aqueous phase (a) and a phase (b) which is immiscible with water, characterised in that:
- the aqueous phase (a) comprises up to 50% by weight, relative to the weight of the phase (a), of trichloroacetic acid; and
- the step (b) immiscible with water comprises perfluorodecalin.

According to an embodiment, the biphasic composition according to this invention comprises:
- 55-95% by weight, preferably 60-85% by weight, of the aqueous phase (a) and
- 5-45% by weight, preferably 15-40% by weight, of the phase (b) which is immiscible with water,
wherein the percentages of the phase (a) and of the phase (b) are referred to the total weight of the biphasic composition.

According to an embodiment, the biphasic composition according to the invention consists of the aqueous phase (a) and the phase (b) which is immiscible with water.

Preferably, the aqueous phase (a) comprises from 1% to 50% by weight, more preferably from 3% to 35% by weight, even more preferably from 5% to 25% by weight, for example from 10% to 20% by weight, with respect to the weight of the phase (a), of trichloroacetic acid.

Trichloroacetic acid has a keratolytic and exfoliating action, due to the denaturation of skin proteins (keratin). Topical treatment with trichloroacetic acid is known to increase the brightness of the skin, favour the discolouring of skin spots, stimulate the synthesis of collagen and hyaluronic acid, favour the absorbance of other active ingredients applied simultaneously or subsequently and stimulate cell turnover.

Optionally but preferably, the aqueous phase (a) also comprises from 0.01% to 5% by weight, preferably from 0.05% to 5% by weight, more preferably from 0.05% to 2% by weight, for example from 0.1% to 2% by weight, with respect to the weight of the phase (a), of a hydrosoluble compound selected between azelaic acid, an organic derivative of azelaic acid, an inorganic derivative of azelaic acid and combinations thereof.

Preferably, the aqueous phase (a) comprises an organic derivative of azelaic acid. More preferably, the derivative of azelaic acid is a amide of azelaic acid, preferably of a formula 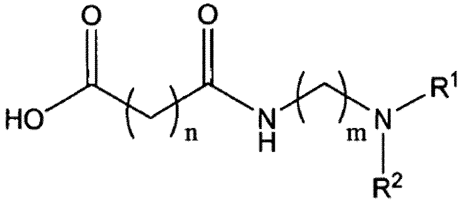 where
n is a whole number between 6 and 10; m is a whole number between 2 and 4; R¹ and R² are independently selected between linear or branched alkyl groups with 1 to 6 carbon atoms.

According to a particularly preferred embodiment, the derivative of azelaic acid is azelamidopropyl dimethyl amine.

Azelaic acid and its derivatives, and in particular azelamidopropyl dimethyl amine, are known to perform a regenerating action of the skin, promoting skin repair and epithelial formation in damaged skin.

The biphasic composition according to any one of the preceding claims, wherein the aqueous phase (a) also comprises up to 20% by weight, preferably from 1% to 10% by weight, relative to the weight of the phase (a) of an alpha-hydroxy acid, preferably having the formula R³R⁴-C(OH)-COOH wherein R³ and R4 are independently selected among H, a straight chain or branched alkyl radical with 1 to 6 carbon atoms, optionally containing heteroatoms, and an aryl or heteroaryl radical with 5 to 12 carbon atoms.

The alpha-hydroxy acid useful for making this invention is preferably selected between glycolic acid, lactic acid, citric acid, tartaric acid, mandelic acid, malic acid and their combinations. Preferably, the alpha-hydroxy acid is mandelic acid.

The alpha-hydroxy acids are chemical exfoliants used in various dermatological and cosmetic compositions.

According to an embodiment, the aqueous phase (a) comprises:
- up to 50% by weight, preferably from 1% to 50% by weight, more preferably from 3% to 35% by weight, even more preferably from 5% to 25% by weight, for example from 10% to 20% by weight, with respect to the weight of the aqueous phase (a), of trichloroacetic acid;
- from 0.01 to 5% by weight, preferably from 0.05% to 5% by weight, more preferably from 0.05% to 2%, for example from 0.1% to 2% by weight, with respect to the weight of the phase (a), of the hydrosoluble compound selected amongst azelaic acid, an organic derivative of azelaic acid, an inorganic derivative of azelaic acid and combinations thereof as described above;
- up to 20% by weight, preferably from 1% to 10% by weight, with respect to the weight of the aqueous phase (a), of an alpha-hydroxy acid as described above; and
- water up to 100%.

Optionally but preferably, the aqueous phase (a) also comprises up to 5% by weight relative to the weight of the phase (a), preferably from 0.5 ppm to 3% by weight, more preferably from 0.8 ppm to 1% by weight, of a compound selected from vitamins, derivatives of vitamins, peptides, derivatives of peptides and combinations thereof.

According to a preferred embodiment, the vitamin in the aqueous phase (a) is vitamin B12.

Optionally, but preferably, the aqueous phase may comprise up to 10% by weight of further excipients and additives for cosmetic use, such as viscosifying agents. According to a preferred embodiment, the aqueous phase (a) comprises up to 3% by weight, relative to the weight of the phase (a), of a viscosifying agent, more preferably of a xanthan rubber.

According to an embodiment, the phase (b) immiscible with water consists of perfluorodecalin.

According to a further embodiment, the phase (b) immiscible with water comprises, in addition to the perfluorodecalin, a mixture of perfluorocarbons other than perfluorodecalin.

According to this embodiment, the mixture of perfluorocarbons other than perfluorodecalin comprises or consists of perfluorohexane, perfluoro perhydrophenanthrene and perfluoro dimethylcyclohexane. According to a preferred embodiment, the phase (b) immiscible with water comprises perfluorodecalin in a quantity of between 1% and 50% by weight, preferably between 5% and 20% by weight, more preferably between 10% and 15% by weight, with respect to the weight of the phase (b) immiscible with water.

According to a particularly preferred embodiment, the phase (b) immiscible with water comprises or consists of perfluorohexane, perfluoro perhydrophenanthrene, perfluoro dimethylcyclohexane and perfluorodecalin, wherein the perfluorodecalin is in a quantity of between 1% and 50% by weight, preferably between 5% and 20% by weight, more preferably between 10% and 15% by weight, with respect to the weight of the phase (b).

Perfluorodecalin is a chemical compound for cosmetic use known in the prior art as an "oxygen conveyor".

An oxygen-enriched cellular environment adversely affects the formation of the bacteria responsible for the formation of the papules and pustules present on the skin with acne.

The biphasic composition according to the invention is preferably characterised by a relative positioning of the phases (a) and (b) wherein the aqueous phase (a) is positioned above the phase (b) immiscible with water.

The biphasic composition according to this invention may be prepared using apparatuses known in the prior art and therefore not described further.

The biphasic composition according to the invention may be prepared by adding the aqueous phase (a) to the phase (b) immiscible with water or vice versa, without stirring and optionally in an inert atmosphere.

Preferably, the biphasic composition according to this invention may be prepared with a method comprising the steps of:
(i) providing an aqueous phase (a) as described above;
(ii) providing a phase (b) immiscible with water as described above;
(iii) positioning the aqueous phase (a) on the surface of the phase (b) immiscible with water.

The step (i) is implemented by mixing the components included in the aqueous phase (a).

In step (iii) the aqueous phase (a) is positioned on the surface of phase (b) immiscible with water in such a way as to completely cover the surface, limiting or avoiding contact of the aqueous phase (a) with the air.

The biphasic composition according to the invention may be applied by spreading the biphasic composition on the skin with a spatula or a brush and leaving the biphasic composition in contact with the skin for a time of between 1 and 10 minutes.

Subsequently, the biphasic composition can be removed by cleaning the skin with water.

The perfluorodecalin of phase (b) has a combined and synergic action with the exfoliating action of the trichloroacetic acid present in phase (a), increasing the effectiveness of the biphasic composition according to the invention in the treatment of acne and the skin blemishes correlated with it with respect to the prior art compositions.

In the treatment of acne, the use of the biphasic composition according to the invention therefore allows better performance to be obtained or comparable to the formulations known in the prior art, by applying a smaller quantity of tricloroacetic acid on the skin.

When used on non-acne skin due to its exfoliating effect, the composition according to this invention induces a faster tissue regeneration and promotes the absorbance of the formulations used in the topical treatments applied after exfoliation.

The side effects following application of the composition according to this invention are less than those of the prior art formulations and the tolerability of the composition according to the invention on skin is better than that of the prior art compositions.

The invention is illustrated below by way of some nonlimiting examples.

### EXAMPLES

The capacity to reduce the amount of cytokines in cell cultures of human keratinocytes was measured for the products in question. In particular, production of interleukin-1α (IL-1α) and interleukin-6 (IL-6) has been evaluated. This capacity is thought to make the product a potentially soothing candidate in vivo, which can reduce the process of irritation and reddening of the skin. A preliminary test of cellular vitality was first performed in order to identify the product concentrations on which to conduct the subsequent doses. Cytokine production in the cells was induced using lipopolysaccharide (LPS). A series of cells were maintained in the ground and not treated (NC, negative control), a series was treated with a known anti-inflammatory substance (PC, positive control), a series was treated with the samples under examination at the established concentrations. At the end of the contact time, the dosing of interleukins was performed on the ground, while a second test of cellular vitality was performed on the cells to verify that there were no significant variations in the experimental conditions.

### MATERIALS AND METHODS

### Cell Culture

The test was performed on human keratinocytes (Huker) cultivated in DMEM (Dulbecco's Modified Eagle Medium) containing 10% of foetal bovine serum (FBS) and 1% of antibiotics (penicillin and streptomycin) and incubated under standard culture conditions (37°C, 5% CO2).

### Cellular Vitality Assessment

Cell viability was assessed by MTT test [3-(4,5-dimethylthiazol-2-yl)-2,5-diphenyltetrazolium bromide], a yellow tetrazolium compound that is reduced by the purple formazan cells. This conversion is caused by NADPH or by the NADH produced by the dehydrogenase in metabolically active cells. The cells were treated with MTT (1 mg/ml) and incubated for 3 hours under standard conditions. At the end of this period the MTT solution was removed and 100 µl of isopropanol was added in each well to dissolve the formazan crystals formed. The absorbance (optical density, OD) has been determined by reading the spectrophotometer at the wavelength of 570 nm.

### Cytokine dosing

In order to stimulate the cytokine production and thus simulate an irritation condition, the cells were stimulated with lipopolysaccharide (LPS). A series of cells were not stimulated in order to evaluate the baseline production of interleukins. Of the cells stimulated with LPS, one series was not treated and maintained in the ground (NC, negative control), one series was treated with a known anti-inflammatory substance (PC, positive control) and one series was treated with the sample under examination at the selected concentrations.

After an overnight incubation period, the content of interleukin in the ground was determined by the ELISA test (Enzyme-Linked Immunosorbent Assay). In an ELISA test a specific antigen for the interleukin to be identified is immobilized on a solid surface (the bottom of a well) to which the dosing means are added. The detection is performed by means of a biotinylated secondary antibody and then caused to react with streptavidin-HRP. The colorimetric reaction is proportional to the quantity of cytokine present. The results are read by means of a spectrophotometer at 450 nm. The values obtained were then interpolated in a standard interleukin curve.

### Preliminary assessment of cellular vitality

The absorbance (OD) measured at 570 nm is proportional to the cellular vitality. The percentages in the table were calculated on the basis of the OD absorbance values at 570 nm and considering the OD of untreated cells as 100%.

### Example 1 and Comparative Example CE2

The compositions of the example 1 according to the invention and of the comparative example CE2 have been prepared as described above. The ingredients are shown in Table 1.

**Table 1**

| | **E1** | **EC2** |
|---|---|---|
| | %weight | %weight |
| Step a) | | |
| Trichloroacetic acid | 8 | 8 |
| Mandelic acid | 4 | 4 |
| Vitamin B12 | 0.0009 | 0.0009 |
| Azelaic acid derivative | 0.01-0.1 | 0.01-0.1 |
| Xanthan gum | 0.1667 | 0.1667 |
| water | qty. enough for 100 | qty. enough for 100 |

| Step b) | | |
|---|---|---|
| perfluorodecalin | 12 | / |
| Mixture of perfluorohexane, perfluoroperhydrophenanthrene and perfluoro dimethylcyclohexane | qty. enough for 100 | / |

**Table 2**

| Concentration | Cellular vitality | |
|---|---|---|
| | E1 | EC2 |
| 0.0078 | 100.95 | 104.50 |
| 0.0156 | 96.03 | 105.17 |
| 0.0313 | 98.25 | 105.81 |
| 0.0625 | 96.32 | 100.56 |
| 0.125 | 97.12 | 97.44 |
| 0.250 | 95.43 | 100.17 |
| 0.500 | 94.46 | 96.59 |
| 1.000 | 90.72 | 98.44 |

Based on the results obtained, it was decided to perform cytokine dosing using concentrations of 1.0, 0.5 and 0.25 mg/ml.

### End of test cellular vitality assessment

The absorbance (OD) measured at 570 nm is proportional to the cellular vitality. The percentages shown in the table were calculated based on OD values at 570 nm and considering the OD of the cells untreated and unstimulated with LPS as 100%. NC = negative control (untreated cells); PC = positive control (cells treated with anti-inflammatory substance).

**Table 3**

| | | Average OD value | Cellular vitality inhibition % |
|---|---|---|---|
| NC | | 1.164 | 0.6 |
| Example E1 | 1.0 mg/ml | 1.106 | 5.7 |
| | 0.5 mg/ml | 1.087 | 7.1 |
| | 0.25 mg/ml | 1.072 | 8.5 |
| EC2 Comp. Example | 1.0 mg/ml | 1.100 | 6.1 |
| | 0.5 mg/ml | 1.178 | 0.0 |
| | 0.25 mg/ml | 1.132 | 3.3 |
| PC | | 1.141 | 2.5 |

The treatment with LPS and with the tested sample do not result in significant variations in cellular vitality.

### IL-1α dosing

The absorbance measured at 450 nm is directly proportional to the quantity of interleukin produced by the cells. In order to calculate the interleukin concentrations, the OD values obtained were interpolated in a standard interleukin curve. The percentages were calculated with respect to the unstimulated and untreated cells (LPS-). The values are expressed as means ± standard deviation.

The statistical data processing was performed by the Student's t-test. Values of p<0.05 are considered significant. NC = untreated control cells; PC = positive control (cells treated with a known anti-inflammatory substance).

**Table 4**

| | | pg/ml | % of LPS- | % Reduction |
|---|---|---|---|---|
| NC | | 76.51 | 117.8 | - |
| Example E1 | 1.0 mg/ml | 65.49 | 100.8 (*#) | 14.4 |
| | 0.5 mg/ml | 66.00 | 101.6(*#) | 13.7 |
| | 0.25 mg/ml | 70.62 | 108.7 | 7.7 |
| EC2 Comp. Example | 1.0 mg/ml | 72.67 | 111.8 | 5.0 |
| | 0.5 mg/ml | 73.44 | 113.0 | 4.0 |
| | 0.25 mg/ml | 73.44 | 113.0 | 4.0 |
| PC | | 62.15 | 95.7 | 18.8 |

| | | | | |
|---|---|---|---|---|
| * p<0.05 vs NC; #p<0.05 vs Sample B. | | | | |

The composition of example E1 is able to significantly reduce the production of IL-1α at the tested concentrations of 1.0 and 0.5 mg/ml (reduction of 14.4% and 13.7%, respectively, compared to untreated cells). The product of the comparative example CE2, at the concentrations tested, does not determine a significant reduction in IL-1α.

### Dose IL-6

The absorbance measured at 450 nm is directly proportional to the quantity of interleukin produced by the cells. In order to calculate the interleukin concentrations, the OD values obtained were interpolated in a standard interleukin curve. The percentages were calculated with respect to the unstimulated and untreated cells (LPS-). The values are expressed as means ± standard deviation.

The statistical data processing was performed by the Student's t-test. Values of p<0.05 are considered significant. NC = untreated control cells; PC = positive control (cells treated with a known anti-inflammatory substance).

**Table 5**

| | | pg/ml | % of LPS- | % Reduction |
|---|---|---|---|---|
| NC | | 764.84 | 397.8 | - |
| Example E1 | 1.0 mg/ml | 786.61 | 409.1 | 0.0 |
| | 0.5 mg/ml | 824.45 | 427.3 | 0.0 |
| | 0.25 mg/ml | 777.42 | 404.4 | 0.0 |
| EC2 Comp. Example | 1.0 mg/ml | 754.03 | 392.2 | 1.4 |
| | 0.5 mg/ml | 804.84 | 418.6 | 0.0 |
| | 0.25 mg/ml | 766.13 | 398.5 | 0.0 |
| PC | | 264.35 | 137.5 | 65.4 |

The products, at the tested concentrations, do not determine a significant variation of IL-6.

Overall, the results obtained demonstrate a soothing *in vitro* effect of the biphasic composition of the E1 example. The tested product significantly reduces the amount of IL-1α in human keratinocyte cultures where an irritation condition has been simulated. This effect is significantly greater than that obtained with the product of the comparative example CE2.

### Example 3 and Comparative Example CE4

### In vivo assessment of keratolytic efficacy and cellular renewal

The in vivo test was conducted on 20 volunteers.

### APPLICATION

The biphasic composition of example E3 was applied In a randomized and standardised manner to one half of the face without makeup and thoroughly cleaned and the product of the comparative example CE4 was applied to the other half. The compositions tested in examples E3 and CE4 are shown in Table 6.

3 minutes after the application, a water-soaked gauze was applied to each half of the face and the products were carefully removed.

Immediately after treatment and then for two consecutive days, the subjects applied a moisturizing cream on their face.

### TIME POINTS

T0: assessment before applying the peeling
T1: immediately after peeling
T2: 48 hours after peeling

### INSTRUMENTATION ASSESSMENT

For the *texture index* of the skin: Antera 3D^{®} at T0 and T2.

For the exfoliation: D-Squame^{®} to evaluate the quantity of corneocytes removed by the treatment at T0 and T2. The instrumentation acquisitions were made in a standardised manner at a temperature of 22°C±2°C and a relative humidity of 50%.

### DERMATOLOGICAL EVALUATION

Performed by a dermatologist using a clinical score.

T0: Prior to the start of the observation test of baseline skin conditions of volunteers assessing redness and dryness.

T1: Immediately after the peeling, redness, dryness and any signs of discomfort were observed by the subjects.

### DATA ANALYSIS

Data statistically analysed with T test (α = 0.05).

**Table 5**

| | **E3** | **CE4** |
|---|---|---|
| | %weight | %weight |
| Step a) | | |
| Trichloroacetic acid | 8 | 12 |
| Mandelic acid | 4 | 4 |
| Vitamin B12 | 0.0009 | 0.0009 |
| Azelaic acid derivative | 0.01-0.1 | 0.01-0.1 |
| Xanthan gum | 0.1667 | 0.1667 |
| water | qty. enough for 100 | qty. enough for 100 |

| Step b) | | |
|---|---|---|
| perfluorodecalin | 12 | / |
| Mixture of perfluorohexane, perfluoroperhydrophenanthren e and perfluoro dimethylcyclohexane | qty. enough for 100 | / |

**Table 6**

| | Example E3 | | Comparative Example CE4 | |
|---|---|---|---|---|
| | Instrum. | Derm. | Instrum. | Derm. |
| | T0 vs T2 | T0 vs T1 | T0 vs T2 | T0 vs T1 |
| Texture index | - 14%(*) | - | -12% (*) | - |
| Keratolytic effect | - 12%(*) | - | -8%(*) | - |
| Redness | - | +55% (*) | - | +48% (*) |
| Dryness | - | +10%(*) | - | 0% |

| | | | | |
|---|---|---|---|---|
| (*) sign. | | | | |

For the texture index and keratolytic effect, a decrease in the parameter indicates an improvement.

## Claims

1. A biphasic composition for topical use comprising an aqueous phase (a) and a water-immiscible phase (b), **characterised in that**
- the aqueous phase (a) comprises up to 50% by weight, relative to the weight of phase (a), of trichloroacetic acid; and
- the water-immiscible phase (b) comprises perfluorodecalin.

2. The biphasic composition according to claim 1, wherein the aqueous phase (a) further comprises from 0.01% to 5% by weight, preferably from 0.05% to 2% by weight, with respect to the weight of phase (a), of a hydro-soluble compound selected from azelaic acid, organic azelaic acid derivative, inorganic azelaic acid derivative and combinations thereof, wherein the organic or inorganic azelaic acid derivative is an azelaic acid amide.

3. The biphasic composition according to claim 1 or 2, wherein the azelaic acid derivative is an azelaic acid amide, having formula 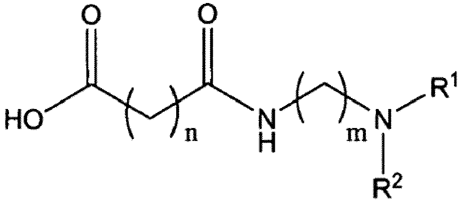 where
n is an integer from 6 to 10; m is an integer from 2 to 4; R1 and R2 are independently selected from linear or branched alkyl groups with 1 to 6 carbon atoms, preferably the azelaic acid derivative is azelamidopropyl dimethylamine.

4. The biphasic composition according to any one of the preceding claims, wherein the aqueous phase (a) also comprises up to 20% by weight, relative to the weight of phase (a), of an alpha-hydroxy acid, preferably having the formula R³R⁴C(OH)-COOH wherein R³ and R⁴ are independently selected from H, a linear or branched alkyl group with 1 to 6 carbon atoms, optionally containing heteroatoms, and an aryl or heteroaryl radical with 5 to 12 carbon atoms.

5. The biphasic composition according to any one of the preceding claims, wherein the aqueous phase (a) further comprises up to 5% by weight relative to the weight of phase (a), preferably from 0.5 ppm to 3% by weight, more preferably from 0.8 ppm to 1% by weight, of a compound selected from vitamins, derivatives of vitamins, peptides and combinations thereof.

6. The biphasic composition according to any one of the preceding claims, wherein the water-immiscible phase (b) comprises a mixture of perfluorocarbons other than perfluorodecalin.

7. The biphasic composition according to claim 6, wherein the mixture of perfluorocarbons other than perfluorodecalin comprises perfluorohexene, perfluoro perhydrophenanthrene and perfluoro dimethylcyclohexane.

8. The biphasic composition according to any one of the preceding claims, wherein the water-immiscible phase (b) comprises perfluorodecalin in a quantity of between 1% and 50% by weight, preferably between 5% and 20% by weight, more preferably between 10% and 15% by weight, with respect to the weight of the phase (b).

9. The biphasic composition according to any one of the preceding claims, wherein the aqueous phase (a) is positioned above the water-immiscible phase (b).

10. The biphasic composition according to any one of the preceding claims, wherein the aqueous phase (a) comprises from 1% to 50% by weight, preferably from 3% to 35% by weight, more preferably from 5% to 25% by weight, for example from 10% to 20% by weight, of trichloroacetic acid, with respect to the weight of the phase (a).

11. The biphasic composition according to any one of the preceding claims comprising
- 55-95% by weight, preferably 60-85% by weight, of the aqueous phase (a) and
- 5-45% by weight, preferably 15-40% by weight, of the water-immiscible phase (b),
wherein the percentages of phase (a) and of phase (b) are referred to the total weight of the biphasic composition.

12. The biphasic composition according to any one of the preceding claims for use in the treatment of acne and related skin blemishes.

13. A method for preparing the biphasic composition according to any one of claims 1 to 11 comprising the steps of:
(i) providing an aqueous phase (a) as described in any one of claims 1 to 5;
(ii) providing a water-immiscible phase (b) as described in any one of claims 6 to 8;
(iii) positioning the aqueous phase (a) on the surface of the phase (b) immiscible with water.

## Patentansprüche

1. Zweiphasige Zusammensetzung zur topischen Verwendung, umfassend eine wässrige Phase (a) und eine mit Wasser nicht mischbare Phase (b), **dadurch gekennzeichnet, dass**
- die wässrige Phase (a) bis zu 50 Gew.-%, bezogen auf das Gewicht der Phase (a), Trichloressigsäure umfasst und
- die mit Wasser nicht mischbare Phase (b) Perfluordecalin umfasst.

2. Zweiphasige Zusammensetzung nach Anspruch 1, wobei die wässrige Phase (a) ferner 0,01 bis 5 Gew.-%, vorzugsweise 0,05 bis 2 Gew.-%, bezogen auf das Gewicht der Phase (a), einer wasserlöslichen Verbindung, ausgewählt aus Azelainsäure, organischen Azelainsäurederivaten, anorganischen Azelainsäurederivaten und Kombinationen davon, umfasst, wobei das organische oder anorganische Azelainsäurederivat ein Azelainsäureamid ist.

3. Zweiphasige Zusammensetzung nach Anspruch 1 oder 2, wobei es sich bei dem Azelainsäurederivat um ein Azelainsäureamid handelt, aufweisend die Formel 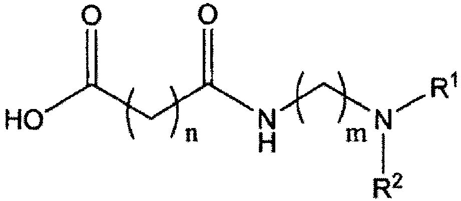 wobei
n eine ganze Zahl von 6 bis 10 ist, m eine ganze Zahl von 2 bis 4 ist, R1 und R2 unabhängig voneinander aus linearen oder verzweigten Alkylgruppen mit 1 bis 6 Kohlenstoffatomen ausgewählt sind und das Azelainsäurederivat vorzugsweise Azelamidopropyldimethylamin ist.

4. Zweiphasige Zusammensetzung nach einem der vorhergehenden Ansprüche, wobei die wässrige Phase (a) auch bis zu 20 Gew.-%, bezogen auf das Gewicht von Phase (a), einer alpha-Hydroxysäure umfasst, vorzugsweise aufweisend die Formel R³R⁴-C(OH)-COOH, wobei R³ und R⁴ unabhängig voneinander aus H, einer linearen oder verzweigten Alkylgruppe mit 1 bis 6 Kohlenstoffatomen, die optional Heteroatome enthält, und einem Aryl- oder Heteroarylrest mit 5 bis 12 Kohlenstoffatomen ausgewählt sind.

5. Zweiphasige Zusammensetzung nach einem der vorhergehenden Ansprüche, wobei die wässrige Phase (a) ferner bis zu 5 Gew.-%, bezogen auf das Gewicht von Phase (a), vorzugsweise von 0,5 ppm bis 3 Gew.-%, noch bevorzugter von 0,8 ppm bis 1 Gew.-%, einer Verbindung umfasst, die aus Vitaminen, Derivaten von Vitaminen, Peptiden und Kombinationen davon ausgewählt ist.

6. Zweiphasige Zusammensetzung nach einem der vorhergehenden Ansprüche, wobei die mit Wasser nicht mischbare Phase (b) ein Gemisch aus anderen Perfluorkohlenstoffen als Perfluordecalin umfasst.

7. Zweiphasige Zusammensetzung nach Anspruch 6, wobei das Gemisch aus anderen Perfluorkohlenstoffen als Perfluordecalin Perfluorhexen, Perfluorperhydrophenanthren und Perfluordimethylcyclohexan umfasst.

8. Zweiphasige Zusammensetzung nach einem der vorhergehenden Ansprüche, wobei die mit Wasser nicht mischbare Phase (b) Perfluordecalin in einer Menge zwischen 1 und 50 Gew.-%, vorzugsweise zwischen 5 und 20 Gew.-%, besonders bevorzugt zwischen 10 und 15 Gew.-%, bezogen auf das Gewicht der Phase (b), umfasst.

9. Zweiphasige Zusammensetzung nach einem der vorhergehenden Ansprüche, wobei die wässrige Phase (a) über der mit Wasser nicht mischbaren Phase (b) positioniert ist.

10. Zweiphasige Zusammensetzung nach einem der vorhergehenden Ansprüche, wobei die wässrige Phase (a) 1 bis 50 Gew.-%, vorzugsweise 3 bis 35 Gew.-%, noch bevorzugter 5 bis 25 Gew.-%, beispielsweise 10 bis 20 Gew.-%, Trichloressigsäure, bezogen auf das Gewicht der Phase (a), umfasst.

11. Zweiphasige Zusammensetzung nach einem der vorhergehenden Ansprüche, umfassend
- 55-95 Gew.-%, vorzugsweise 60-85 Gew.-%, der wässrigen Phase (a) und
- 5-45 Gew.-%, vorzugsweise 15-40 Gew.-%, der mit Wasser nicht mischbaren Phase (b),
wobei die Prozentsätze von Phase (a) und von Phase (b) auf das Gesamtgewicht der zweiphasigen Zusammensetzung bezogen sind.

12. Zweiphasige Zusammensetzung nach einem der vorhergehenden Ansprüche zur Verwendung bei der Behandlung von Akne und verwandten Hautunreinheiten.

13. Verfahren zur Herstellung der zweiphasigen Zusammensetzung nach einem der Ansprüche 1 bis 11, umfassend die folgenden Schritte:
(i) Bereitstellen einer wässrigen Phase (a) nach einem der Ansprüche 1 bis 5;
(ii) Bereitstellen einer mit Wasser nicht mischbaren Phase (b) nach einem der Ansprüche 6 bis 8;
(iii) Positionieren der wässrigen Phase (a) auf der Oberfläche der mit Wasser nicht mischbaren Phase (b).

## Revendications

1. Composition biphasique pour utilisation topique comprenant une phase aqueuse (a) et une phase non miscible à l'eau (b), **caractérisée en ce que**
- la phase aqueuse (a) comprend jusqu'à 50 % en poids, par rapport au poids de la phase (a), d'acide trichloracétique ; et
- la phase non miscible à l'eau (b) comprend de la perfluorodécaline.

2. Composition biphasique selon la revendication 1, dans laquelle la phase aqueuse (a) comprend en outre de 0,01 % à 5 % en poids, de préférence de 0,05 % à 2 % en poids, par rapport au poids de la phase (a), d'un composé hydrosoluble choisi parmi l'acide azélaïque, un dérivé organique de l'acide azélaïque, un dérivé inorganique de l'acide azélaïque et leurs combinaisons, dans laquelle le dérivé organique ou inorganique de l'acide azélaïque est un amide d'acide azélaïque.

3. Composition biphasique selon la revendication 1 ou 2, dans laquelle le dérivé de l'acide azélaïque est un amide d'acide azélaïque ayant la formule 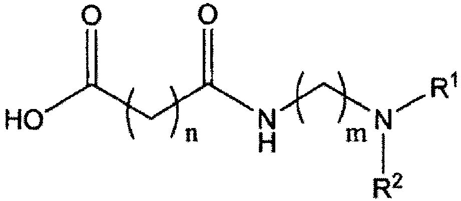 où
n est un nombre entier de 6 à 10 ; m est un nombre entier de 2 à 4 ; R1 et R2 sont indépendamment choisis parmi des groupes alkyles linéaires ou ramifiés ayant de 1 à 6 atomes de carbone ; de préférence, le dérivé de l'acide azélaïque est l'azélamidopropyl diméthylamine.

4. Composition biphasique selon l'une quelconque des revendications précédentes, dans laquelle la phase aqueuse (a) comprend également jusqu'à 20 % en poids, par rapport au poids de la phase (a), d'un alpha-hydroxyacide, ayant de préférence la formule R³R⁴-C(OH)-COOH dans laquelle R³ et R⁴ sont indépendamment choisis parmi H, un groupe alkyle linéaire ou ramifié ayant de 1 à 6 atomes de carbone, contenant optionnellement des hétéroatomes, et un radical aryle ou hétéroaryle ayant de 5 à 12 atomes de carbone.

5. Composition biphasique selon l'une quelconque des revendications précédentes, dans laquelle la phase aqueuse (a) comprend en outre jusqu'à 5 % en poids par rapport au poids de la phase (a), de préférence de 0,5 ppm à 3 % en poids, plus préférentiellement de 0,8 ppm à 1 % en poids, d'un composé choisi parmi les vitamines, les dérivés de vitamines, les peptides et leurs combinaisons.

6. Composition biphasique selon l'une quelconque des revendications précédentes, dans laquelle la phase non miscible à l'eau (b) comprend un mélange de perfluorocarbones autres que la perfluorodécaline.

7. Composition biphasique selon la revendication 6, dans laquelle le mélange de perfluorocarbones autres que la perfluorodécaline comprend du perfluorohexène, du perfluoro perhydrophénanthrène et du perfluoro diméthylcyclohexane.

8. Composition biphasique selon l'une quelconque des revendications précédentes, dans laquelle la phase non miscible à l'eau (b) comprend de la perfluorodécaline en une quantité comprise entre 1 % et 50 % en poids, de préférence entre 5 % et 20 % en poids, plus préférentiellement entre 10 % et 15 % en poids, par rapport au poids de la phase (b).

9. Composition biphasique selon l'une quelconque des revendications précédentes, dans laquelle la phase aqueuse (a) est placée au-dessus de la phase non miscible à l'eau (b).

10. Composition biphasique selon l'une quelconque des revendications précédentes, dans laquelle la phase aqueuse (a) comprend de 1 % à 50 % en poids, de préférence de 3 % à 35 % en poids, plus préférentiellement de 5 % à 25 % en poids, par exemple de 10 % à 20 % en poids, d'acide trichloracétique, par rapport au poids de la phase (a).

11. Composition biphasique selon l'une quelconque des revendications précédentes comprenant
- 55-95 % en poids, de préférence 60-85 % en poids, de la phase aqueuse (a) et
- 5-45 % en poids, de préférence 15-40 % en poids, de la phase non miscible à l'eau (b),
dans laquelle les pourcentages de la phase (a) et de la phase (b) sont rapportés au poids total de la composition biphasique.

12. Composition biphasique selon l'une quelconque des revendications précédentes, destinée à être utilisée pour le traitement de l'acné et des imperfections cutanées connexes.

13. Procédé de préparation de la composition biphasique selon l'une quelconque des revendications de 1 à **11,** comprenant les étapes consistant à :
(i) fournir une phase aqueuse (a) telle que décrite dans l'une quelconque des revendications de 1 à 5 ;
(ii) fournir une phase non miscible à l'eau (b) telle que décrite dans l'une quelconque des revendications de 6 à 8 ;
(iii) placer la phase aqueuse (a) sur la surface de la phase (b) non miscible à l'eau.
